(19) Europäisches Patentamt
European Patent Office
Office européen des brevets

(11) **EP 2 685 256 A1**

(12) **EUROPEAN PATENT APPLICATION**
published in accordance with Art. 153(4) EPC

(43) Date of publication:
**15.01.2014 Bulletin 2014/03**

(51) Int Cl.:
***G01N 30/88*** (2006.01)

(21) Application number: **12755343.6**

(22) Date of filing: **12.03.2012**

(86) International application number:
**PCT/JP2012/056302**

(87) International publication number:
**WO 2012/121408 (13.09.2012 Gazette 2012/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.03.2011 JP 2011073486**

(71) Applicants:
• **CellSeed Inc.
Tokyo 162-0056 (JP)**
• **Tokyo Women's Medical University
Tokyo 162-8666 (JP)**
• **Kanazawa, Hideko
Sagamihara-shi, Kanagawa 252-0318 (JP)**

(72) Inventors:
• **KANAZAWA, Hideko
Kanagawa 252-0318 (JP)**

• **NAGASE, Kenichi
Tokyo 162-8666 (JP)**
• **KOBAYASHI, Jun
Tokyo 162-8666 (JP)**
• **KIKUCHI, Akihiko
Nagareyama-shi, Chiba 270-0156 (JP)**
• **AKIYAMA, Yoshikatsu
Tokyo 162-8666 (JP)**
• **OKANO, Teruo
Tokyo 162-8666 (JP)**

(74) Representative: **HOFFMANN EITLE
Patent- und Rechtsanwälte
Arabellastrasse 4
81925 München (DE)**

(54) **TEMPERATURE-RESPONSIVE MONOLITHIC POROUS BODY, METHOD FOR PRODUCING SAME, AND TEMPERATURE-RESPONSIVE CHROMATOGRAPHY METHOD USING SAME**

(57) A temperature responsive monolithic porous material is obtained that comprises a polymer having a hydration ability that changes in a temperature range of 0 to 80°C and being immobilized to a surface of the porous material at a high density by binding an atom transfer radical polymerization initiator to a surface of the porous material, and inducing a growth reaction of a polymer, having a hydration ability that changes in a temperature range of 0 to 80°C, from the initiator using an atom transfer radical process under a presence of a catalyst.

Figure 1

**Description**

TECHNICAL FIELD

The present invention relates to a temperature responsive monolithic silica porous material, a production method thereof, and a temperature responsive chromatography process using the same, in which the temperature responsive monolithic silica porous material is a filler for liquid chromatography that is performed by using an external signal, the temperature, to control the interaction of usable materials, such as medicines, biological materials (protein, DNA, glycolipids, etc.) and cells, with the surface of a solid.

BACKGROUND ART

The high-performance liquid chromatography (HPLC) provides various combinations of the mobile phase liquid and the stationary phase and a variety of such combinations can be selected according to the reagent, which led to the recent use of HPLC to separate and/or refine various substances. However, the conventional chromatography induces the interaction between the solute contained in the mobile phase and the surface of the stationary phase mainly by changing the solvent in the mobile phase, and leaving the surface structure of the stationary phase unchanged. In an example of HPLC, which is used in many fields, the normal phase column that uses carriers of silica gel and the like as the stationary phase uses a mobile phase of organic solvents, such as hexane, acetonitrile, and chloroform, and the reversed phase column that uses the silica gel derivative separated by the aqueous system uses organic solvents, such as methanol and acetonitrile. Additionally, the ion exchange chromatography, whose stationary phase consists of an anion exchanger or a cation exchanger, is characterized by a separation of substances inducing a change in the external ion concentration or type. The recent rapid development of genetic engineering and the like evoked expectations for a wide application of bioactive peptides, proteins, DNAs and the like in different fields including medicine, and their separation/refinement are becoming an important problem to be solved. In particular, a need is developing for a technology to separate/refine bioactive substances without inhibiting their activity. However, the organic solvent, acid, alkali, and surfactants used in the conventional mobile phase inhibit the substance's activity and also act as contaminants, so a system modification is called for. The need to prevent environmental contamination by these substances also necessitates a separation/refinement system that does not use these substances.

Various studies have been conducted under such circumstances. In particular, the technology in Patent Document 1 constitutes the base technology. The document describes a technology of cultivating cells at the maximum critical solution temperature or lower or the minimum critical solution temperature or higher on a cell culture substrate that is coated on the surface with a polymer having a maximum or minimum critical solution temperature against water of 0 to 80°C, then peeling off the cultivated cell by altering the temperature to the maximum critical solution temperature or higher or the minimum critical solution temperature or lower. The document provides the first example of using the temperature responsive polymer in the cell culture material in the field of biomedicine, but it should be noted that in the adhesion of cells to the substrate surface, cells secrete adhesive protein and adheres to the substrate via that protein. This means that in the phenomenon of cells being peeled off from the substrate surface, the adhesive protein secreted from the cells is also peeled off. As a matter of fact, when the cells obtained by the technology is reinoculated, or grafted to a tissue, those cells that have been peeled off from the substrate adhere well to the substrate or the tissue. This indicates that the peeled-off cells retain the adhesive protein that they had secreted during cultivation. That is, this technology is exactly the concept of the temperature responsive chromatography technology described in the present invention, which is to detach the adsorbed protein by changing temperatures.

Under the above circumstances, Patent Document 2 considers binding subjects to conventional chromatography carriers, namely silica gel or polymer gel. However, specific details were unclear, since there is no result (separation chart) in the Examples of separated solutes when such carrier was used, and there is no description of specific substances that can be separated using the carrier or the specific problems to be solved.

On the other hand, Patent Document 3 shows actual examples of immobilizing temperature responsive polymers to the silica gel surface, and using that carrier to separate various steroids and lymphocytes. It clearly shows that the characteristics of temperature responsive polymers immobilized to the surface of the silica gel carrier induces the separation of various steroids and lymphocytes. However, the method of immobilizing temperature responsive polymers, shown in the document, only allowed polymers to be immobilized to the substrate surface at a maximum density of about 0.7 mg/m$^2$ due to the high volume of the polymer, and the chromatography carrier could only exhibit limited features. There was a demand for an innovative technology that improves the prior art by a detailed design of immobilizing the temperature responsive polymer to the substrate surface.

Under such circumstances, Patent Document 4 discloses using the atom transfer radical polymerization process to binding temperature responsive polymers to the silica gel surface at a high density, and an example of separation using such carrier is shown. However, the width of the separation peak is large in all Examples, and further technical improvement was required for its use as a separation method of useful, biologically active substances.

Further, a column of monolithic silica is attracting attention as a new chromatography carrier to replace conventional silica beads, in the recent analytical chemistry field. The monolithic silica column is composed of a single network having a three-dimensional network structure, and the passing of the mobile phase through such network structure enables the liquid delivery pressure in the chromatography analysis to be reduced and the diffusion length until the interaction of the solute to be shortened, so that various substances can be analyzed at a high sensitivity (Non-patent Documents 1, 2, 3). However, no one thought of binding temperature responsive polymers in such high-performance monolith columns, and it was not easy to analogically predict whether the feature of a coated temperature responsive polymer will be expressed by such combination, or how such feature will be expressed.

CITATION LIST

PATENT DOCUMENT

    Patent Document 1: Japanese examined patent publication No. H06-104061
    Patent Document 2: Japanese unexamined patent publication No. Hows-133947
    Patent Document 3: Japanese unexamined patent publication No. H07-318551
    Patent Document 4: Japanese unexamined patent publication No. 2007-69193

NON-PATENT DOCUMENT

    Non-Patent Document 1: N. Tanaka, H. Kobayashi, K. Nakanishi, H. Minakuchi and N. Ishizuka, Anal. Chem., 73, 420-429 (2001)
    Non-Patent Document 2: O. Nunez, K. Nakanishi and N. Tanaka, J. Chromatogr. A, 1191, 231-252 (2008)
    Non-Patent Document 3: R. E. Paproski, J. Cooley and C. A. Lucy, Analyst, 131, 422-428 (2006)

SUMMARY OF INVENTION

TECHNICAL PROBLEM

The present invention was performed to solve the above problems of the conventional art. That is, the present invention aims to provide a novel temperature responsive monolithic porous material based on a completely different idea from the conventional art. Additionally, the present invention aims to provide a production method of such carriers. Further, the invention aims to provide a temperature responsive chromatography process using such carriers.

SOLUTION TO PROBLEM

The present inventors conducted research and development to solve the above problem by analyzing the above problem from various angles. Consequently, the present inventors discovered to their surprise that the use of a temperature responsive monolithic porous material having a polymer, whose hydration ability changes in a temperature range of 0 to 80°C, bound to the porous material surface at a high density of 0.01 molecular chain/nm$^2$ or higher leads to a significant improvement in the degree of change against a temperature change, compared to the chromatography carrier obtained by the conventional art. The technology of the present invention could not have been predicted from the conventional art, and it is expected to develop into a new chromatography system unprecedented by any conventional art. The present invention was completed based on such insights.

That is, the present invention provides a temperature responsive monolithic porous material comprising a polymer having a hydration ability that changes in a temperature range of 0 to 80°C and being bound to a substrate surface at a high density of 0.01 molecular chain/nm$^2$ or higher.

In addition, the present invention provides a production method of a temperature responsive monolithic porous material comprising the steps of: binding an atom transfer radical polymerization initiator to a substrate surface; and inducing a growth reaction of a polymer, having a hydration ability that changes in a temperature range of 0 to 80°C, from the initiator using an atom transfer radical process under a presence of a catalyst.

Further, the present invention provides a temperature responsive chromatography process using the temperature responsive monolithic porous material.

ADVANTAGEOUS EFFECTS OF INVENTION

A new separation system is proposed based on the temperature responsive monolithic porous material of the present invention. A wide variety of peptides, proteins and cells can be separated by using this system.

BRIEF DESCRIPTION OF DRAWINGS

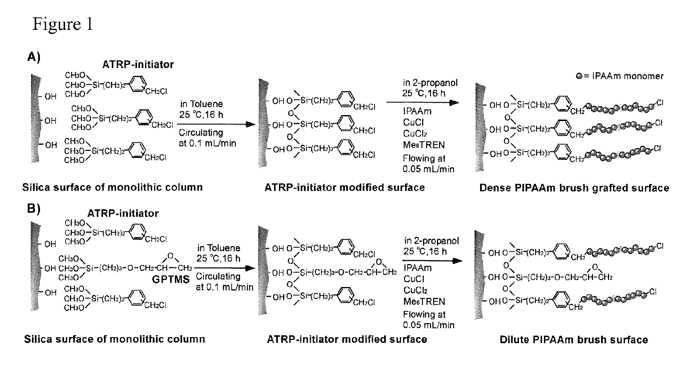

[Figure 1] Figure 1 is a diagram showing a production method of poly(N-isopropylacrylamide) brush-modified monolithic silica using the atom transfer radical polymerization process of Example 1. In the drawing, (A) shows a high density PIPAAm-modified monolithic silica, (B) represents a PIPAAm-modified monolithic silica with a reduced PIPAAm density.

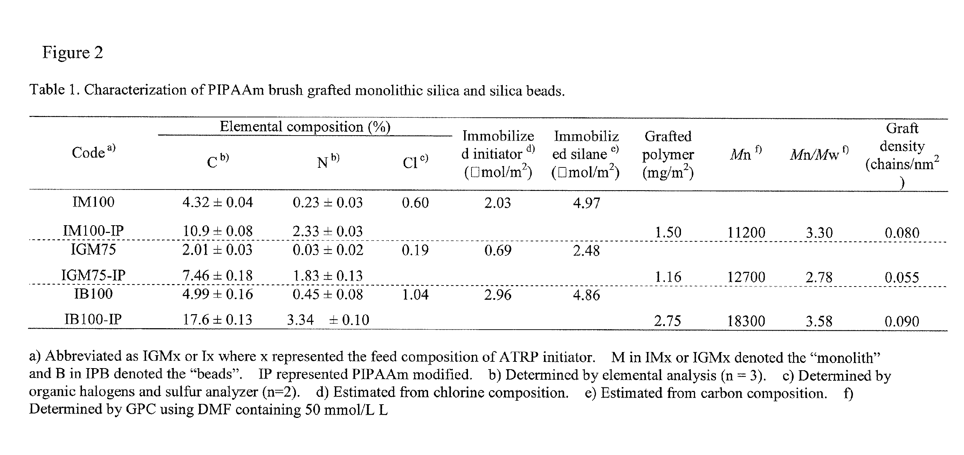

[Figure 2] Figure 2 is a diagram showing the elemental analysis result of the samples obtained in Example 1 (IM100, IGM75), and Comparative Example 1 (IB100).

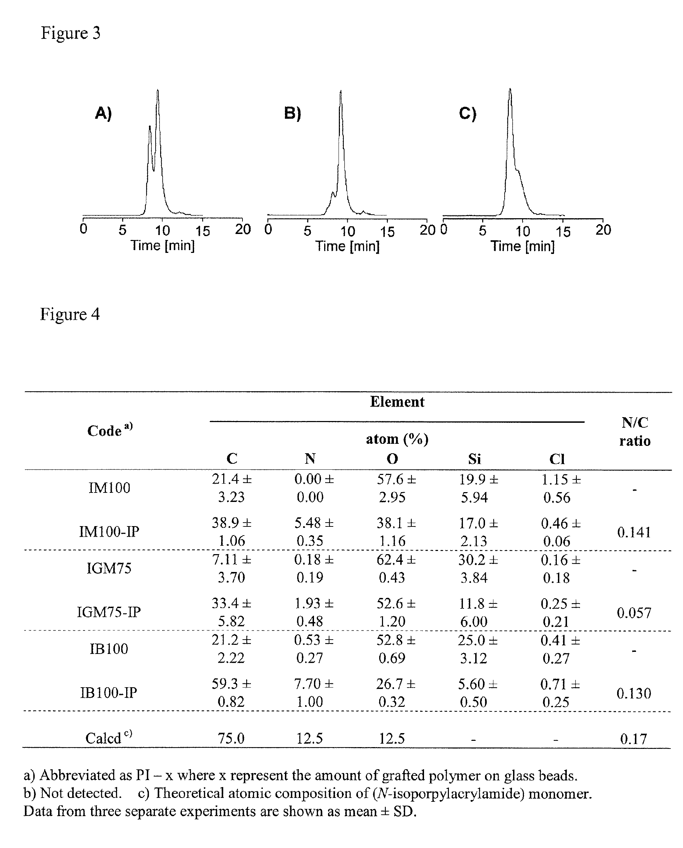

[Figure 3] Figure 3 is a diagram showing the analysis result of GPC performed in Example 1 (IM100, IGM75), and Comparative Example 1 (IB100). (A) is a result for IM100, (B) for IGM75, and (C) for IB 100. GPC was performed using a mobile phase of DMF containing 50 mmol/L LiCl.
[Figure 4] Figure 4 is the result of a measurement using XPS of the element composition of the surface of the samples obtained in Example 1 (IM100, IGM75), and Comparative Example 1 (IB100).

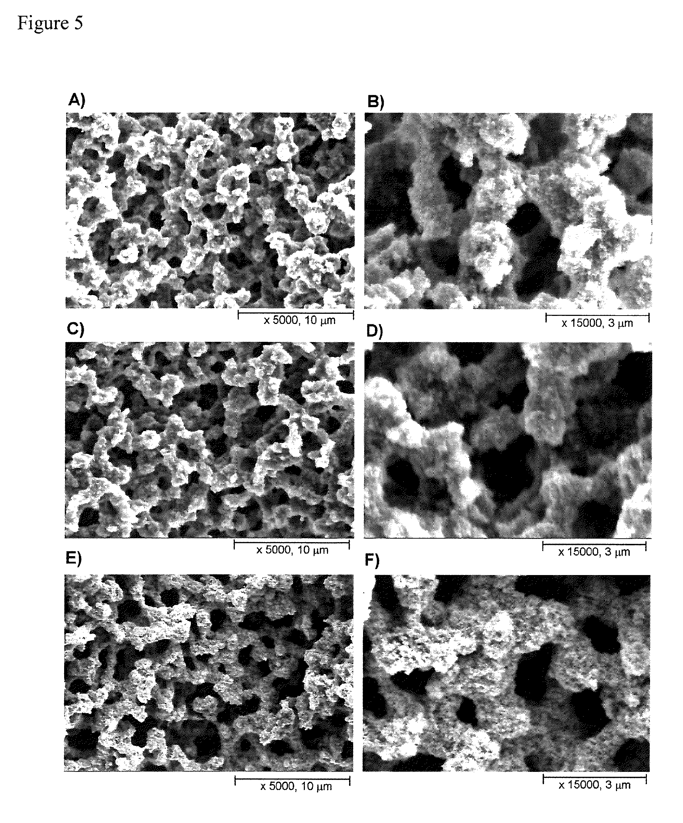

[Figure 5] Figure 5 is a result of observing the poly(N-isopropylacrylamide) brush-modified monolithic silica obtained in Example 1 with a scanning electron microscope. In the drawing, (A)(B) show a PIPAAm-modified monolithic silica modified at a high density, (C)(D) show monolithic silica having a reduced PIPAAm modification density, and (E)(F) show a non-modified monolithic silica.

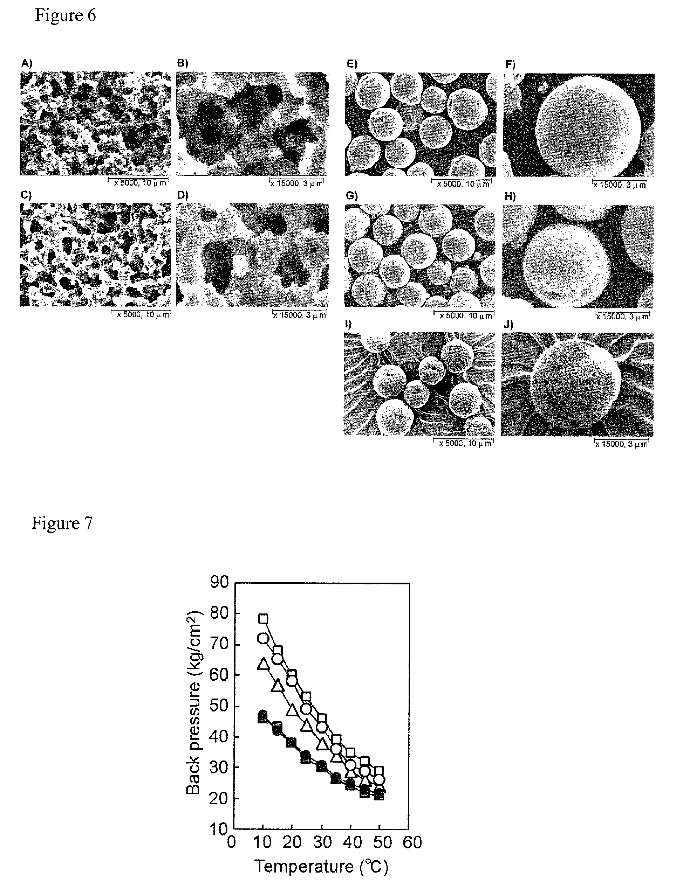

[Figure 6] Figure 6 is a result of observing the samples obtained in Example 1 and Comparative Example 1 with a scanning electron microscope. In the drawing, (A)(B) show a monolithic silica having an initiator fixed to it, (C)(D) show a monolithic silica having PIPAAm fixed to it, and (E)(F) show silica beads having PIPAAm fixed to it, and (G)(H) show silica beads having an initiator fixed to it, and (I)(J) show a non-modified monolithic silica.
[Figure 7] Figure 7 is a result in each column of a temperature-induced change in the mobile-phase liquid-delivery-pressure, using the samples obtained in Example 1 and Comparative Example 1. In the drawing, (○) shows a high density PIPAAm-modified monolithic silica, (△) shows a monolithic silica having a reduced PIPAAm modification density, (□) shows a high density, PIPAAm-modified silica beads, (●) shows a non-modified monolithic silica, (■) shows a non-modified monolithic silica.

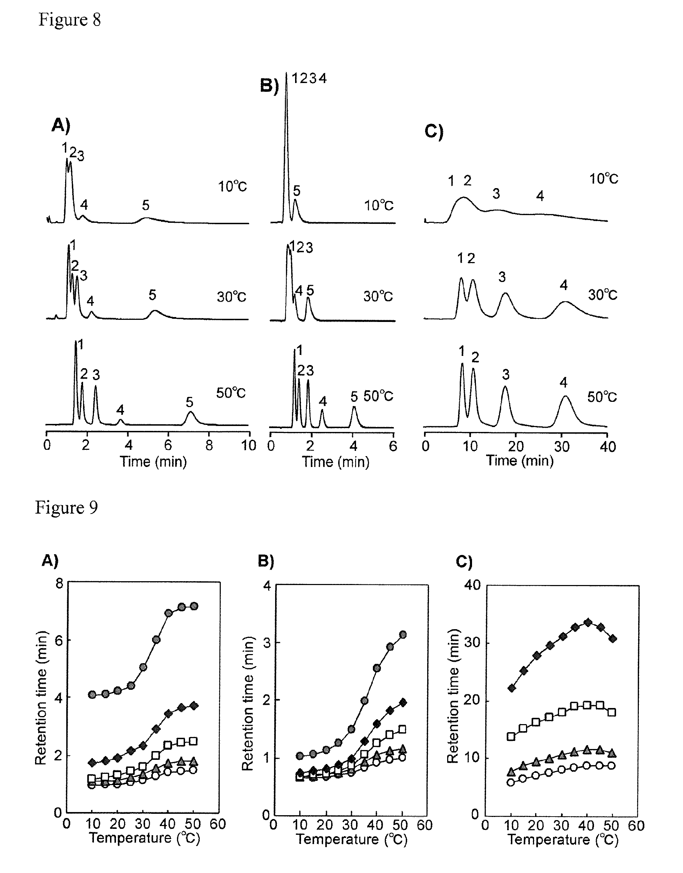

[Figure 8] Figure 8 is a drawing showing the result of separating hydrophobic steroids in each column, using the samples obtained in Example 1 and Comparative Example 1. In the drawing, (A) shows a high density PIPAAm-modified monolithic silica, (B) shows a monolithic silica having a reduced PIPAAm modification density, and (C) shows a high density PIPAAm-modified silica beads. The mobile phase was Milli-Q water, and the flow rate was 1.0 mL/min. In each graph, Peak 1 is hydrocortisone, Peak 2 is prednisolone, Peak 3 is dexamethasone, Peak 4 is hydrocortisone acetate, and Peak 5 is testosterone.
[Figure 9] Figure 9 is a drawing showing the result of separating hydrophobic steroids in each column, using the samples obtained in Example 1 and Comparative Example 1. The temperature-induced change in the retention time was confirmed. In the drawing, (A) shows a high density PIPAAm-modified monolithic silica, (B) shows a monolithic silica having a reduced PIPAAm modification density, and (C) shows a high density PIPAAm-modified silica beads. ((○) Hydrocortisone, (▲) prednisolone, (□) dexamethasone, (♦) hydrocortisone acetate, (●) testosterone)

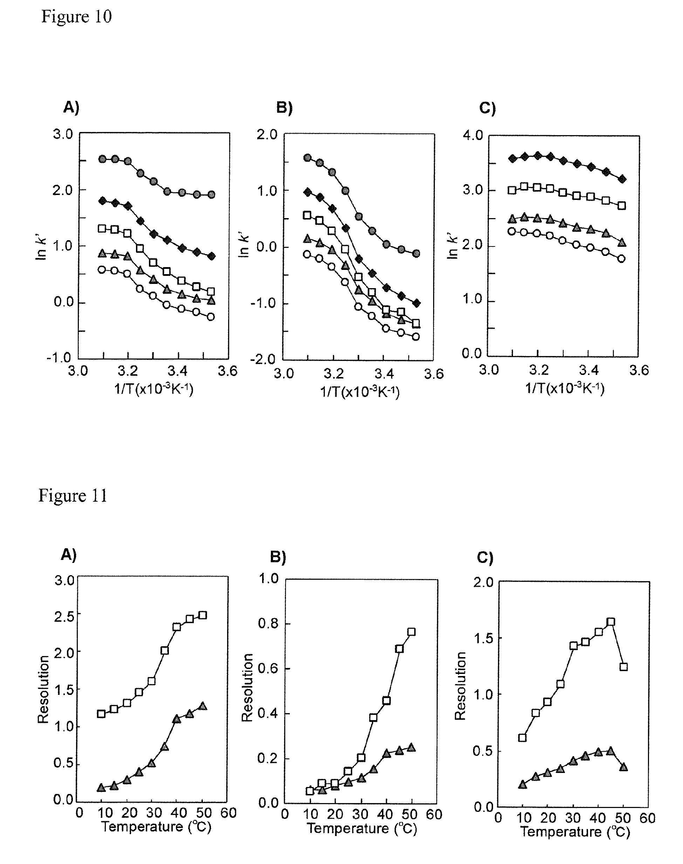

[Figure 10] Figure 10 is a drawing showing the result of separating hydrophobic steroids in each column, using the samples obtained in Example 1 and Comparative Example 1. The temperature-induced retention time behavior in each column was analyzed using the van't Hoff plot. In the drawing, (A) shows a high density PIPAAm-modified monolithic silica, (B) shows a monolithic silica having a reduced PIPAAm modification density, and (C) shows high density PIPAAm-modified silica beads. ((○) hydrocortisone, (A) prednisolone, (□) dexamethasone, (♦) hydrocortisone acetate, (●) testosterone)
[Figure 11] Figure 11 is a drawing showing the result of separating hydrophobic steroids in each column, using the samples obtained in Example 1 and Comparative Example 1. The temperature-induced solute separability in each column was analyzed. In the drawing, (A) shows a high density PIPAAm-modified monolithic silica, (B) shows a monolithic silica having a reduced PIPAAm modification density, and (C) shows a high density PIPAAm-modified silica beads. ((○) hydrocortisone, (A) prednisolone, (□) dexamethasone, (♦) hydrocortisone acetate, (●) testosterone)

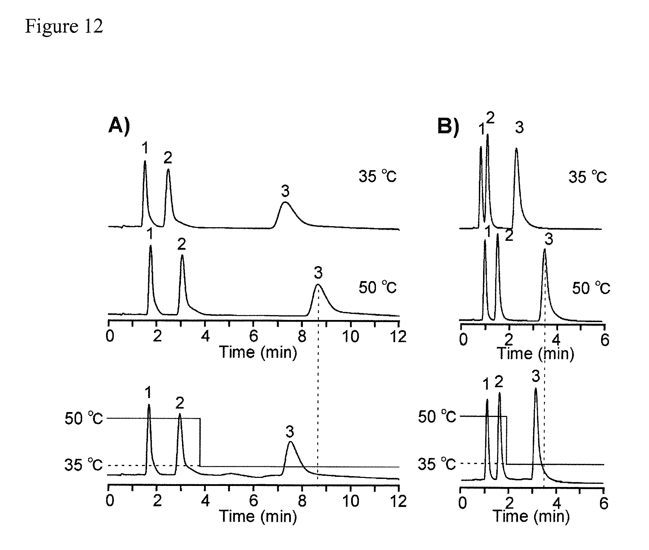

[Figure 12] Figure 11 is a drawing showing the result of separating hydrophobic steroids in each column, using the samples obtained in Example 1 and Comparative Example 1. The level of reduction of the analysis time due to an abrupt change in the temperature was studied. In the drawing, (A) shows a high density PIPAAm-modified monolithic silica, (B) shows a monolithic silica having a reduced PIPAAm modification density, and (C) shows a high density PIPAAm-modified silica beads. In each graph, Peak 1 is hydrocortisone, Peak 2 is dexamethasone, and Peak 3 is testosterone.

DESCRIPTION OF EMBODIMENTS

The present inventors conducted varied studies to satisfy the above demand, and developed a technology for performing separation/refinement and concentration by changing the interaction between a specific matter that is dissolved or dispersed in the mobile phase and the stationary phase surface without changing the mobile phase, which is enabled through a change in the surface structure of the stationary phase caused by altering external conditions including temperature, and thus completed the invention. The object of the present invention is to provide a chromatography method that can perform separation, refinement and concentration by a single aqueous mobile phase and a temperature responsive monolithic porous material, which is a stationary phase used in such chromatography, by changing the external condition, and thereby reversibly changing the surface characteristics of the stationary phase.

An aspect of the present invention is a chromatography method that separates specific materials while retaining an aqueous-based mobile phase by using a temperature responsive monolithic porous material that can change the characteristics of the stationary phase surface by temperature. In addition, the present invention provides a production method of such temperature responsive monolithic porous material. Further, the present invention provides a temperature responsive chromatography process using the above method. That is, the use of the present invention enables separation of biological elements such as peptides, proteins and cells, by raising the external temperature to the critical temperature or above. Accordingly, the complete absence of the use of organic solvents, acids, alkalis, surfactants or other agents prevents generation of contaminants from such materials, and allows the use of the invention in separation similar to an analysis performed by maintaining the features of protein, cells and the like.

In a conventional chromatography process, separation of reagents is difficult when separating/analyzing reagents of various mixed compounds in a single mobile phase, especially when there are multiple reagents with a large difference in the polarity, and the time required for separation is extremely prolonged. Hence, when using such reagents, separation is performed by the solvent gradient process, in which the amount and types of the organic solvent is continuously changed, or the step gradient process, in which the amount and types of the organic solvent is changed in steps. By comparison, the temperature gradient process or the step gradient process of the present invention can achieve the same separation in a single mobile phase by changing the column temperature continuously or in steps instead of using an organic solvent. By adopting this method, it becomes possible to prevent the incorporation of the above contaminants, and to perform separation while maintaining the features of protein and cells. In addition, it becomes possible to separate a desired material in a short time by controlling the temperature.

The present invention is shown in detail below. The porous material used in the present invention is a monolith-type porous material. The material of the porous material is not particularly limited, but examples include silica, polystyrene, or the hybrid type combining them. The porous material of the present invention can be produced according to conventional methods including the sol-gel method, but many are already distributed on the market, and porous materials of silica are especially abundant in variation and preferable. The present invention is practicable according to the material of the product. The conventional high-performance liquid chromatography filled with silica beads tends to increase in the load pressure to the filled silica beads and decrease in the separation a when the flow rate of the mobile phase is increased to shorten the analysis time, improve the separation performance and conduct a large scale analysis. The monolith-type porous material used in the present invention can be used without receiving such load and the separation performance does not decrease. The shape of the monolith porous body is not particularly limited, but a common column size has an inner diameter of 2 mm to 5 mm, and a length of 2 cm to 30 cm. The monolith-type porous material can be larger in size according to the usage, or it can be in a capillary shape to improve the separation ability.

The present invention is a temperature responsive monolithic porous material, in which the polymer having a hydration ability altering in the temperature range of 0 to 80°C, immobilized on the porous material surface at a high density of 0.01 molecular chain/nm$^2$ or higher. The immobilization at a high density induces a significant expression of the polymer property of the polymers immobilized on the carrier surface. The reason is presently unknown, but it is assumed that the phenomenon results from the immobilized polymer existing at a high density on the carrier surface causing a close involvement with the neighboring polymer chains. However, this reason does not limit the technology of the present invention in any way.

The polymer of the present invention having a hydration ability changing in the temperature range of 0 to 80°C includes a polymer having a minimum critical solution temperature (LCST), and a polymer having a maximum critical solution temperature (UCST), and it can be any of a homopolymer, a copolymer or a mixture thereof. An example of such polymer is the polymer in the Japanese Examined Patent Publication No. H06-104061. Specifically, the polymer can be obtained by a simple polymerization or a copolymerization of the following monomers. Monomers that can be used include (meth)acrylamide compounds, N-(or N,N-di)alkyl substituted (meth)acrylamide derivatives, or a vinyl ether derivative, a partially acetylated product of polyvinyl alcohol, and any two of these can be used to form a copolymer. In addition, a copolymer with monomers other than the above monomer, a graft polymer or copolymer of polymers, or a mixture of a polymer and/or a copolymer can be used. Further, bridging in a range that does not damage the inherent property of the polymer is possible. The substance to be separated is a biological substance, and the separation is performed at a temperature in the range of 5°C to 50°C. The polymers to be used under such conditions include poly-N-n-propylacrylamide (the minimum critical solution temperature of a homopolymer is 21 °C), poly-N-n-propylmethacrylamide (the same temperature is 27°C), poly-N-isopropylacrylamide (the same temperature is 32°C), poly-N-isopropylmethacrylamide (the same temperature is 43°C), poly-N-cyclopropylacrylamide (the same temperature is 45°C), poly-N-ethoxyethylacrylamide (the same temperature is 35°C), poly-N-ethoxyethylmethacrylamide (the same temperature is about 45°C), poly-N-tetrahydrofurfurylacrylamide (the same temperature is about 28°C), poly-N-tetrahyrofrufurylmethacrylamide (the same temperature is about 35°C), poly-N,N-ethylmethylacrylamide (the same temperature is about 56°C), poly-N,N-diethylacrylamide (the same temperature is about 32°C).

The hydrophilic polymer used in the present invention can be either a homopolymer or a copolymer. Examples include without being limited thereby, hydrous polymers, such as polyacrylamide, poly-N,N-diethylacrylamide, poly-N,N-dimethylacrylamide, polyethylene oxide, polyacryl acid and its salt, polyhydroxyethylmethacrylate, polyhydroxyethylacrylate, polyvinyl alcohol, polyvinyl pyrrolidone, cellulose, carboxymethyl cellulose.

In the present invention, the above polymers are bound at a high density The level of binding should be 0.01 molecular chain/$nm^2$ or higher, preferably 0.04 molecular chain/$nm^2$ or higher, and more preferably 0.08 molecular chain/$nm^2$ or higher by the number of molecular chain to a unit area. A degree of binding of the polymer to the substrate surface that is 0.01 molecular chain/$nm^2$ or lower merely leads to the expression of individual polymer chain properties similar to the conventional method of binding polymers to substrate surfaces. The method of calculating numerical values that indicate the level of binding is not particularly limited, but an exemplary method of obtaining the values is to prepare, under the same reaction conditions, a polymer that is not bound to the substrate surface to obtain a molecular amount from an analysis of the polymer chain, and a polymer bound to a porous material to obtain the amount of bound-polymer from an elemental analysis, and comparing the two amounts.

The molecular amount of the polymer to be bound is not particularly limited as long as it is high enough to enable a change in the hydration ability to be expressed in the temperature range of 0 to 80°C, but the polymer molecular amount should be 1000 or higher, preferably 2000 or higher, and more preferably 5000 or higher. A molecular amount of 1000 or lower is not desirable, since the excessively low molecular amount prevents the expression of a change in the hydration ability. Also, a molecular amount of 5000 or higher is undesirable, since an excessively high molecular amount of the polymer leads to a high volume of the molecule itself and the temperature responsiveness will drop.

The immobilized-polymer amount on the substrate shown in the present invention should be in the range of 0.2 to 10.0 mg/$m^2$, preferably in the range of 0.4 to 8.0 mg/$m^2$, and more preferably in the range of 0.8 to 6.0 mg/$m^2$. Temperature responsiveness becomes unrecognizable at 0.2 mg/$m^2$ or lower, and a value that is higher than 10.0 mg/$m^2$ is also not desirable, since the high volume of the polymer leads to a decrease in the temperature responsiveness. The immobilized amount can be measured according to conventional methods, such as elemental analysis, or ESCA, but any method can be used. The state of polymers to be immobilized in the present invention is not particularly limited, and includes a straight chain state and a bridged state, but the former straight chain state is desirable to increase responsiveness to temperature and to immobilize the polymer to the substrate surface at a high density.

The present invention includes fixing the above polymer to the monolithic porous material. The method of fixation is not particularly limited, but an example is a method of fixing an atom transfer radical polymerization initiator to a surface of the porous material, and inducing a growth reaction of a polymer, having a hydration ability that changes in a temperature range of 0 to 80°C, from the initiator using an atom transfer radical process under a presence of a catalyst. The initiator to be used in the process is not particularly limited, but examples for the present invention using a substrate of silica or glass may include 2-m/p-chloromethylphenylethyl trimethoxysilane, 2-m/p-chloromethylphenylethyl trichlorosilan, 1-trichlorosilyl-2-m/p-chloromethylphenyl ethane, 2-(4-chlorosulfonylphenyl)ethyl trimethoxysilan, (3-(2-bromoisobutyryl)propyl) dimethylethoxysilane. The expression, "m/p," indicates a meta-form and/or a para-form. Polymer chains are grown from such initiator in the present invention. Such catalysts are not particularly limited, but examples of copper halides ($Cu^{I}X$) include $Cu^{I}Cl$, $Cu^{I}Br$, when an N-alkyl-substituted (meth)acrylamide derivative is selected as the polymer having a changing hydration ability. The ligand complex corresponding to the copper halide is not particularly limited either, but examples include tris(2-(dimethylamino)ethyl)amine ($Me_6$TREN), N,N,N",N"- pentamethyldiethylenetriamine (PMDETA), 1,1,4,7,10,10-hexamethyltriethylenetetraamine (HMTETA), 1,4,8,11-tetramethyl, 1,4,8,11-azacyclotetradecane ($Me_4$Cyclam), bipyridine. Solvents to be used in polymerization are not particularly limited, and include dimethylformaldehyde (DMF). Other conditions including the initiator concentration, copper halide concentration, ligand complex concentration, reaction temperature, and reaction time are not particularly limited, and they can be modified according to the purpose. Further, the reaction liquid can be kept still or refluxed to a porous material, and the latter is more desirable in view of achieving a uniform fixation to the carrier surface.

The shape of a porous material used in the present invention is not particularly limited, and the porous material may be tabular or tubular. In particular, the carrier should be silica gel when the porous material of the present invention is used as a carrier for chromatography. The pore size is not particularly limited, but it should be 3 to 500 nm, preferably 10 to 100 nm, and more preferably 12 to 50 nm. A pore size of 3 nm or lower allows only solutes with an extremely low molecular amount to be separated, and a pore size of 500 nm or higher cuts down the carrier surface area and leads to an extremely bad separation.

The present invention fills the column with the temperature responsive monolithic porous material that has been thus obtained to attach it to a normal liquid chromatography device, and to use it as a liquid chromatography system. The separation in the present invention is affected by the temperature of the carrier that fills the column. The method of applying heat to the carrier is not particularly limited, but exemplary methods include a method of enclosing all or part of the column filled with the carrier with an aluminum block, a bath, a layer of air or a jacket that is set to a predetermined temperature.

The separation method is not particularly limited, but an example includes a method of separating the solute under a given temperature in a column having a temperature responsive monolithic porous material placed therein. The carrier of the present invention changes its surface property according to the temperature. For some substances to be separated, the separation can be induced just by setting the temperature to an appropriate constant temperature.

Another example of a separation method is to confirm in advance the temperature at which the surface property of the porous material changes, and to crossover that temperature when changing the temperature to separate the solute. This method induces a large change in the surface property of the carrier, just by the temperature change, so the time of signal expression (retention time) is expected to vary greatly for some solutes. The most effective method of using the present invention is to perform separation by crossing over the temperature at which the surface property of the porous material changes.

When changing the temperature, the temperature change can be conducted once or periodically for two or more times, or it can be conducted continuously, from when the solute is introduced. The methods can also be combined. The temperature change can be performed manually, or a device that can automatically control temperature by a program can be used.

Or else, another example of a separation method is to use the catch-and-release method of inducing adsorption of the solute on to the obtained temperature responsive monolithic porous material, and subsequently, changing a temperature to change a surface property of a carrier and to release the solute that has been adsorbed. The amount of solute to be adsorbed may go beyond or stay within the amount that the porous material can adsorb. In any case, this method is a method of first adsorbing the solute and then changing the temperature to change the surface property of the carrier and release the adsorbed solute.

Further, two or more temperature responsive monolithic porous materials can be filled in the same column to separate a solute with a temperature change that crosses over the temperature at which the surface property of the carrier changes. When 2 types of carriers are used, 3 areas of different temperature ranges will appear on the carrier surface, and the temperature can be changed by the above method by crossing over the temperatures of the 3 areas. This process can be performed by filling the 2 or more types of temperature responsive monolithic porous materials into 2 or more columns.

Another example of a separation method is a method of using a temperature responsive monolithic porous material, setting the column inlet end temperature and the column outlet end temperature to include or stride over a temperature at which the surface property of the carrier changes, and to separate the solute by implementing a temperature gradient in the column from the inlet end to the outlet end. The method of gradually changing the temperature is not particularly limited, but examples include a method of maintaining the temperature of the entire column by an adequate monitoring of the column inlet end temperature and the column outlet end temperature, and a method of joining aluminum blocks with different temperatures and bringing them in contact with the column.

The present invention can separate the solute with the temperature alone, while keeping the mobile phase fixed. The mobile phase should preferably be 100% aqueous based, but the present invention depends on the property of the polymer immobilized to the surface of the porous material, so the composition of the mobile phase is not particularly limited, and the mobile phase can include a solvent other than water, such as an organic solvent, or its pH can be changed, or it can include salt. The concentration of the solvent can be changed, and the carrier of the present invention can be used in combination with the solvent gradient method. Further, the mobile phase can be 100% organic solvent.

The use of the temperature responsive monolithic porous material of the present invention, and the chromatography process using the same, as shown above, enables separation of medicines and their metabolites, agricultural chemicals, peptides, proteins, and cells. The separation can be achieved by a simple operation of merely changing the temperature in the column.

EXAMPLES

The present invention is explained in detail below based on the Examples, without being limited thereby.

[Example 1]

(Modifying the monolithic silica in the column with a silane coupling agent)

Poly-N-isopropylacrylamide-modified monolithic silica column (PIPAAm, poly-N-isopropylacrylamide may be referred to as PIPAAm) was obtained by the process of Figure 1. The ATRP initiator (m-chloromethylphenylethyl trimethoxysilane and/or p-chloromethylphenylethyl trimethoxysilane) was modified by the following process. The monolithic silica column (Product name: Monobis, Serial No.: 3250H30SI) was kept still at 75% humidity for 18 hours. M/p-chloromethylphenylethyl trimethoxysilane of 6 mL was dissolved in 14 mL of dehydrated toluene. Then the solution was subjected to reflux in the column at 0.1 mL/min for 16 hours using an HPLC pump. Afterwards, toluene and acetone were passed through the column after reaction for washing, and the column was dried under reduced pressure in an oven of 110°C. Then, to produce a PIPAAm-modified surface with reduced density, an ATRP initiator (4.5 mL) and glycidylpropyl trimethoxysilane (1.36 mL) were dissolved in 14 mL of toluene solution to prepare a mixed solution, and the HPLC pump was used to subject the solution to reflux in the column at 0.1 mL/min for 16 hours, using the HPLC pump. Then, toluene and acetone were passed through the column after reaction for washing, and the column was dried under reduced pressure in an oven of 110°C.

<PIPAAm modification of monolithic silica in the column>

The PIPAAm-brush-modified monolithic silica was produced by the following method. IPAAm (14.6 g, 129 mmol) was dissolved in 85.6 mL of 2-propanole, then, it was bubbled in nitrogen gas for 60 minutes to remove dissolved oxygen. CuCl (168 mg, 1.70 mmol), $CuCl_2$ (23.0 mg, 0.171 mmol), $Me_6$TREN (0.44 g, 1.91 mmol) were added under nitrogen atmosphere, and mixed for 20 minutes to form $CuCl/CuCl_2/Me_6$TREN. The monomer solution, ATRP initiator-modified silica column, and the HPLC pump were placed in the glove box. Pressure reduction and nitrogen gas encapsulation were repeated 3 times to remove oxygen in the glove box, and the solution was passed through the column at 0.05 mL/min for 16 hours. Then, acetone and 2-propanole were passed through the column for washing and dried under reduced pressure at 50°C.

<Physical property assessment of ATRP Initiator, PIPAAm-modified monolithic silica column>

The rod of monolithic silica modified by ATRP initiator was assessed in terms of its properties as follows. A measurement was performed by the trace halogen analysis. The ATRP initiator was obtained by the following formula.

$$\text{Formula 1}$$

$$\frac{\%Cl}{\%Cl(calcd) \times \left(1 - \%Cl / \%Cl(calcd)\right) \times S}$$

Note that %Cl stands for the weight percentage of chlorine, %Cl (calcd) stands for the weight percentage of the chlorine of the initiator, and S is the surface area. The silane coupling agent immobilized to the surface was obtained by the following formula.

Formula 2

$$\frac{\%C_s}{\%C_s(calcd) \times \left(1 - \%C_s / \%C_s(calcd)\right) \times S}$$

Note that $\%C_s$ stands for the weight percentage of carbon, $\%C_s$ (calcd) stands for the weight percentage of carbon in the silane coupling agent. The amount of the modifying PIPAAm was obtained by the following formula.

Formula 3

$$\frac{\%C_p}{\%C_P(calcd) \times \left(1 - \%C_p / \%C_p(calcd) - \%C_s / \%C_s(calcd)\right) \times S}$$

Note that $\%C_P$ stands for the weight percentage of carbon, $\%C_P$ (calcd) stands for the weight percentage of carbon in PIPAAm.

The molecular amount of PIPAAm for modifying monolithic silica was obtained by the following formula. PIPAAm-modified monolithic silica was immersed for 3 hours in the hydrofluoric acid, and then, neutralized by sodium carbonate, followed by dialysis using a permeable membrane to refine the modifying PIPAAm. Then, PIPAAm was recovered by freeze-drying, and the molecular amount and the distribution of the molecular amount were measured by GPC. The obtained molecular amount was obtained using the following formula.

Formula 4

$$\frac{m_C \cdot N_A}{M_n}$$

Note that $M_c$ is an amount of the modifying PIPAAm ($g/m^2$), $N_A$ is an Avogadro's number, $M_n$ is a number average molecular amount. The element composition on the surface was measured by XPS. The surface was observed by SEM.

<Retention behavior of the bioactive substance>

Five types of hydrophobic steroids including hydrocortisone, prednisolone, dexamethasone, hydrocortisone acetate, testosterone, in an amount of 0.217 mg/mL, were dissolved in the Mill-Q water/ethanol mixed solution. The PIPAAm-modified monolithic silica column was connected to the HPLC system, and Milli-Q water was passed through at 1.0 mL/min. The hydrophobic steroid was detected at 254 nm. The following solution was used to analyze the retention behavior of steroid.

Formula 5

$$\frac{t_R - t_0}{t_0}$$

Note that $t_R$ is the retention time of hydrophobic steroids and $t_0$ is the retention time of uracil that does not interact with PIPAAm.

[Comparative Example 1]

<Modification of silica beads by the silane coupling agent>

The ATRP initiator (m/p-chloromethylphenylethyl trimethoxysilane)-modified silica beads was obtained by keeping the silica beads still at 60% humidity, then reacting a toluene solution of ATRP initiator of 53.4 mmol/L with the silica beads for 16 hours. Then, the reacted product was washed with toluene and acetone and dried under reduced pressure at 110°C.

<PIPAAm modification of monolithic silica in the column>

The PIPAAm-modified silica beads was prepared as follows. The ATRP initiator-modified silica beads was reacted with 2-propanole solution having IPAAAm and CuCl/CuCl$_2$/Me$_6$TREN added thereto for 16 hours. Then, the reacted product was washed with acetone, methanol, EDTA solution, and Milli-Q water, and dried under reduced pressure at 50°C.

<Physical property assessment of an ATRP initiator, PIPAAm-modified silica beads>

The silica beads modified with ATRP initiator was assessed in terms of its properties as follows. A measurement was performed by the trace halogen analysis. The ATRP initiator was obtained by the following formula.

Formula 6

$$\frac{\%Cl}{\%Cl(calcd) \times \left(1 - \%Cl / \%Cl(calcd)\right) \times S}$$

Note that %Cl stands for the weight percentage of chlorine, %Cl (calcd) stands for the weight percentage of the chlorine of the initiator, and S is the surface area. The silane coupling agent bound to the surface was obtained by the following formula.

Formula 7

$$\frac{\%C_s}{\%C_s(calcd) \times \left(1 - \%C_s / \%C_s(calcd)\right) \times S}$$

Note that %C$_s$ stands for the weight percentage of carbon, %C$_s$ (calcd) stands for the weight percentage of carbon in the silane coupling agent. The amount of the modifying PIPAAm was obtained by the following formula.

Formula 8

$$\frac{\%C_p}{\%C_P(calcd)\times\left(1-\%C_p/\%C_p(calcd)-\%C_s/\%C_s(calcd)\right)\times S}$$

Note that $\%C_P$ stands for the weight percentage of carbon, $\%C_P$ (calcd) stands for the weight percentage of carbon in PIPAAm.

Formula 9

$$\frac{m_C \cdot N_A}{M_n}$$

Note that $m_c$ is an amount of modifying PIPAAm (g/m$^2$), $N_A$ is an Avogadro's number, $M_n$ is a number average molecular amount. The element composition on the surface was measured by XPS. The surface observation was measured by SEM.

<Retention Behavior of Bioactive Substances>

The PIPAAm-modified silica beads was filled in a column (4.6 mm i.d. x 50 mm). Five types of hydrophobic steroids including hydrocortisone, prednisolone, dexamethasone, hydrocortisone acetate, testosterone, in an amount of 0.217 mg/mL, were dissolved in the Mill-Q water/ethanol mixed solution. The PIPAAm-modified silica beads column was connected to the HPLC system, and Milli-Q water was passed through at 1.0 mL/min. The hydrophobic steroid was detected at 254 nm. The following solution was used to analyze the retention behavior of steroid.

Formula 10

$$\frac{t_R - t_0}{t_0}$$

Note that $t_R$ is the retention time of hydrophobic steroids and $t_0$ is the retention time of uracil that does not interact with PIPAAm.
The results of Example 1 and Comparative Example 1 are compared below.

<Physical Property Analysis of Monolithic silica Column>

The ATRP initiator-modified monolithic silica and the PIPAAm-modified monolithic silica was subjected to elemental analysis. The surface modified by a mixture of the initiator and glycidylpropyl trimethoxysilane, as well as the surface produced by modifying the former surface with PIPAAm were similarly assessed. The result of elemental analysis is shown in Figure 2. The amount of the fixed-initiator obtained by the composition of chlorine elements indicated a small value compared to the amount of the fixed-initiator obtained from the carbon element. This can be considered as resulting from the difference in the sensitivity of elemental analysis. The ATRP initiator amount obtained from the carbon on the surface of monolithic silica column is the same value as that modifying silica beads, which shows that a reaction on the surface of the monolithic silica column is possible by the conditions of keeping still at 75% humidity, an ATRP-initiator-containing toluene solution with a concentration of 30%, and a 0.1 mL/min reflux.

The amount of chlorine elements was larger on the surface prepared only with an ATRP initiator than the surface prepared with a mixture of the ATRP initiator and glycidylpropyl trimethoxysilane. The amount of chlorine element on the surface can be reduced by competitively bonding glycidylpropyl trimethoxysilane.

The PIPAAm-modified monolithic silica and the PIPAAm-modified silica beads were prepared by surface initiator ATRP. The amount of carbon elements increased after reacting ATRP, so it became evident that passing the ATRP reaction solution through the monolithic silica column can modify the surface of the monolithic silica column by PIPAAm.

To measure the modifying PIPAAm chain length and the modifying PIPAAm density, the silica in the PIPAAm-modified monolithic silica and the PIPAAm-modified silica beads was dissolved by the hydrofluoric acid to release the modifying PIPAAm for measurement using GPC. The GPC chart is shown in Figure 3. A hybridization of two narrow peaks was confirmed in the GPC chart. The reason for this is considered as the difference in the polymerization rate of the polymers in the monolithic silica surface and within the pores, or within the monolithic silica beads and the pores, during the modification of the polymer surface, and the termination of polymer modification by the polymer saturating in the 30 nm pore. The density of the modifying PIPAAm was small when glycidylpropyl trimethoxysilane was reacted on the surface. This shows that the modifying PIPAAm density can be controlled by controlling the initiator density.

The elemental composition of the surface was confirmed with XPS, and it was found that the PIPAAm modification by ATRP produces a large proportion of N, and the value of N/C approaches 0.17 (Figure 4). Further, the peak derived from C=O not seen in the initiator-modified silica was confirmed after modification by PIPAAm. Hence, modification by PIPAAm was confirmed.

When the PIPAAm-modified silica and the non-modified monolithic silica were compared using an SEM image, there was no difference in the micropore. This indicated that the flow channel of the mobile phase is not plugged by modification by PIPAAm (Figures 5, 6).

Further, when the pressures of PIPAAm-modified silica and the unmodified silica at each temperature were confirmed, it was found that in the PIPAAm-modified monolithic silica, the pressure drops suddenly as the temperature increases (Figure 7). This is due to the contraction of the modifying PIPAAm by dehydration.

<Elution Behavior of the Hydrophobic Steroid>

Figure 8 shows the elution behavior of steroids at each temperature. The PIPAAm monolith-modified silica column showed a significantly fast elution time (about 1/5) in comparison to the elution behavior of the PIPAAm-modified silica beads. This is due to the significantly fast linear velocity of the mobile phase of the PIPAAm-modified silica, and the short diffusion length of the solute dissolved in the mobile phase and the PIPAAm modifying the monolithic silica, in the monolithic silica. Further, the elution time was further reduced when the density of PIPAAm modifying the monolithic silica was reduced. This is due to the reduction of the amount of the modifying PIPAAm leading to a weakened hydrophobic interaction with the solute.

In the plot of the retention time and temperature of Figure 9, the PIPAAm monolith column showed a behavior of a sudden change in the phase transfer temperature, and the PIPAAm-modified silica showed a gradual increase in the retention time, which decreased from the maximum point of 40°C. This result is considered the feature of a monolith column. That is, when the modification is performed on a monolith, the retention behavior differed even though the modification structure was the same.

PIPAAm-modified monolithic silica column showed a large change in the retention behavior around LCST in the analysis by the Van't Hoff plot in Figure 10. On the other hand, the change in the retention behavior was small in the PIPAAm-modified silica beads.

Figure 11 shows a change in the level of separation in the hydrophobic steroid. The PIPAAm-modified monolithic silica showed a higher level of separation than the PIPAAm-modified silica beads. Hence, the PIPAAm-modified monolithic silica can be considered as a carrier exhibiting a higher level of separation in a shorter analysis time.

Figure 12 shows the chromatogram of a temperature step gradient of a column temperature dropping abruptly from 50°C to 35°C. This showed that an analysis time (retention time) that is short can be further shortened by changing the column temperature abruptly.

As shown above, the PIPAAm-modified monolithic silica is a chromatographic carrier that requires only a short analysis time for analysis, and the analysis time can be controlled freely by temperature.

INDUSTRIAL APPLICABILITY

The present invention provides a temperature responsive monolithic porous material having a polymer, which changes its hydration ability in the temperature range of 0 to 80°C, immobilized at a high density on the substrate surface. The degree of change in the substrate surface due to the change in the temperature is largely enhanced by the use of the carrier. This leads to an easy separation operation and an improved efficiency in the separation process. There is a strong expectancy for its use with objects to be separated including a wide range of peptides, proteins, and cells. Hence, the present invention is extremely advantageous in the field of medicine, pharmacy, analytical chemistry and biology.

**Claims**

1. A temperature responsive monolithic porous material comprising a polymer having a hydration ability that changes in a temperature range of 0 to 80°C and being bound to a surface of the porous material at a density of 0.01 molecular chain/nm$^2$ or higher.

2. The temperature responsive monolithic porous material of Claim 1, wherein the porous material consists of silica.

3. The temperature responsive monolithic porous material according to either Claim 1 or 2, wherein an amount of bound-polymer on the surface of the porous material is 0.2 to 10.0 mg/m$^2$.

4. The temperature responsive monolithic porous material according to any one of Claims 1 to 3, wherein a polymer molecular chain is non-crosslinked.

5. The temperature responsive monolithic porous material according to any one of Claims 1 to 4, wherein the polymer is one or a plurality of poly-N-substituted acrylamide derivative, poly-N-substituted methacrylamide derivative, their copolymer, polyvinylmethyl ether, a partially acetylated polyvinyl alcohol.

6. The temperature responsive monolithic porous material according to any one of Claims 1 to 5, wherein the polymer is poly-N-isopropylacrylamide.

7. The temperature responsive monolithic porous material according to either Claim 5 or 6, wherein the polymer is a copolymer containing a hydrophilic molecule, a hydrophobic molecule and an ionic molecule in a polymer molecular chain, at a range that retains a feature of the hydration ability to change in a temperature range of 0 to 80°C.

8. The temperature responsive monolithic porous material according to any one of Claims 1 to 7, wherein a shape of the porous material is tabular or tubular.

9. A production method of a temperature responsive monolithic porous material comprising the steps of:

binding an atom transfer radical polymerization initiator to a surface of the porous material; and
inducing a growth reaction of a polymer, having a hydration ability that changes in a temperature range of 0 to 80°C, from the initiator using an atom transfer radical process under a presence of a catalyst.

10. The production method according to Claim 9, wherein the porous material consists of silica.

11. The production method according to either Claim 9 or 10, wherein the atom transfer radical polymerization initiator is 2-m-chloromethylphenylethyl trimethoxysilane and/or 2-p-chloromethylphenylethyl trimethoxysilane.

12. The production method according to any one of Claims 9 to 11, wherein the atom transfer radical polymerization initiator is bound at a high density at a rate of 0.01 molecular chain/nm$^2$ or higher.

13. The production method according to any one of Claims 9 to 12, wherein a polymerization catalyst includes copper chloride as a copper halide and tris(2-(dimethylamino)ethyl)amine as a ligand complex.

14. The production method according to any one of Claims 9 to 13, wherein an amount of bound-polymer on the surface of the porous material is 0.2 to 10.0 mg/m$^2$.

15. The production method according to any one of Claims 9 to 14, wherein a polymer molecular chain is non-crosslinked.

16. The production method according to any one of Claims 9 to 15, wherein the polymer is one or a plurality of poly-N-substituted acrylamide derivative, poly-N-substituted methacrylamide derivative, their copolymer, polyvinylmethyl ether, a partially acetylated polyvinyl alcohol.

**17.** The production method according to any one of Claims 9 to 16, wherein the porous material is silica gel particles, a glass plate or glass particles.

**18.** A temperature responsive chromatography process comprising a step of separating or concentrating a specific object by striding over a temperature at which a surface property of the temperature responsive monolithic porous material changes when changing temperatures according to Claims 1 to 8.

**19.** The temperature responsive chromatography process according to Claim 18 comprising the steps of inducing adsorption of the specific object on the temperature responsive monolithic porous material of Claims 1 to 8, and subsequently, changing a temperature to change a surface property of a carrier and to release the specific object that has been adsorbed.

**20.** The temperature responsive chromatography process according to either Claim 18 or 19 comprising a step of separating the specific object while changing temperatures in a range of temperatures striding over a temperature that changes a surface property of a carrier by filling two or more temperature responsive monolithic porous materials according to Claims 1 to 8 in a same column.

**21.** The temperature responsive chromatography process according to any one of Claims 18 to 20 comprising a step of separating the specific object using a temperature responsive monolithic porous material according to Claims 1 to 8 and setting a column inlet end temperature and a column outlet end temperature to temperatures striding over a temperature that changes a surface property of a carrier to create a temperature gradient from an inlet end to an outlet end of a column.

**22.** The temperature responsive chromatography process according to any one of Claims 18 to 21, wherein a mobile phase is aqueous.

**23.** The temperature responsive chromatography process according to Claims 18 to 22, wherein the specific object is a medicine or its metabolite, an agricultural chemical, peptide, protein and a cell.

Figure 1

Figure 2

Table 1. Characterization of PIPAAm brush grafted monolithic silica and silica beads.

| Code [a] | Elemental composition (%) | | | Immobilized initiator [d] ($\square$mol/m$^2$) | Immobilized silane [e] ($\square$mol/m$^2$) | Grafted polymer (mg/m$^2$) | $Mn$ [f] | $Mn/Mw$ [f] | Graft density (chains/nm$^2$) |
| | C [b] | N [b] | Cl [c] | | | | | | |
|---|---|---|---|---|---|---|---|---|---|
| IM100 | 4.32 ± 0.04 | 0.23 ± 0.03 | 0.60 | 2.03 | 4.97 | | | | |
| IM100-IP | 10.9 ± 0.08 | 2.33 ± 0.03 | | | | 1.50 | 11200 | 3.30 | 0.080 |
| IGM75 | 2.01 ± 0.03 | 0.03 ± 0.02 | 0.19 | 0.69 | 2.48 | | | | |
| IGM75-IP | 7.46 ± 0.18 | 1.83 ± 0.13 | | | | 1.16 | 12700 | 2.78 | 0.055 |
| IB100 | 4.99 ± 0.16 | 0.45 ± 0.08 | 1.04 | 2.96 | 4.86 | | | | |
| IB100-IP | 17.6 ± 0.13 | 3.34 ± 0.10 | | | | 2.75 | 18300 | 3.58 | 0.090 |

a) Abbreviated as IGMx or Ix where x represented the feed composition of ATRP initiator.   M in IMx or IGMx denoted the "monolith" and B in IPB denoted the "beads".   IP represented PIPAAm modified.   b) Determined by elemental analysis (n = 3).   c) Determined by organic halogens and sulfur analyzer (n=2).   d) Estimated from chlorine composition.   e) Estimated from carbon composition.   f) Determined by GPC using DMF containing 50 mmol/L L

Figure 3

Figure 4

| Code [a] | Element | | | | | N/C ratio |
|---|---|---|---|---|---|---|
| | atom (%) | | | | | |
| | **C** | **N** | **O** | **Si** | **Cl** | |
| IM100 | 21.4 ± 3.23 | 0.00 ± 0.00 | 57.6 ± 2.95 | 19.9 ± 5.94 | 1.15 ± 0.56 | - |
| IM100-IP | 38.9 ± 1.06 | 5.48 ± 0.35 | 38.1 ± 1.16 | 17.0 ± 2.13 | 0.46 ± 0.06 | 0.141 |
| IGM75 | 7.11 ± 3.70 | 0.18 ± 0.19 | 62.4 ± 0.43 | 30.2 ± 3.84 | 0.16 ± 0.18 | - |
| IGM75-IP | 33.4 ± 5.82 | 1.93 ± 0.48 | 52.6 ± 1.20 | 11.8 ± 6.00 | 0.25 ± 0.21 | 0.057 |
| IB100 | 21.2 ± 2.22 | 0.53 ± 0.27 | 52.8 ± 0.69 | 25.0 ± 3.12 | 0.41 ± 0.27 | - |
| IB100-IP | 59.3 ± 0.82 | 7.70 ± 1.00 | 26.7 ± 0.32 | 5.60 ± 0.50 | 0.71 ± 0.25 | 0.130 |
| Calcd [c] | 75.0 | 12.5 | 12.5 | - | - | 0.17 |

a) Abbreviated as PI – x where x represent the amount of grafted polymer on glass beads.
b) Not detected.    c) Theoretical atomic composition of (N-isoporpylacrylamide) monomer.
Data from three separate experiments are shown as mean ± SD.

Figure 5

A)

x 5000, 10 μm

B)

x 15000, 3 μm

C)

x 5000, 10 μm

D)

x 15000, 3 μm

E)

x 5000, 10 μm

F)

x 15000, 3 μm

Figure 6

Figure 7

Figure 8

Figure 9

Figure 10

Figure 11

Figure 12

## INTERNATIONAL SEARCH REPORT

| International application No. |
|---|
| PCT/JP2012/056302 |

| A. CLASSIFICATION OF SUBJECT MATTER |
|---|
| *G01N30/88*(2006.01)i |

According to International Patent Classification (IPC) or to both national classification and IPC

| B. FIELDS SEARCHED |
|---|

Minimum documentation searched (classification system followed by classification symbols)
G01N30/88

Documentation searched other than minimum documentation to the extent that such documents are included in the fields searched

| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X | JP 2007-69193 A  (Mitsuo OKANO),<br>22 March 2007 (22.03.2007),<br>claims; paragraphs [0021] to [0022]<br>(Family: none) | 1-23 |
| A | JP 2002-119854 A  (Amersham Biosciences Kabushiki Kaisha),<br>23 April 2002 (23.04.2002),<br>claim 8; paragraphs [0011] to [0013]<br>& US 2004/0039177 A1    & EP 1347831 A<br>& WO 2002/030564 A1    & AU 9425301 A<br>& CA 2423589 A | 1 |

| ☐ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 May, 2012 (11.05.12) | 22 May, 2012 (22.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06104061 B
- JP HOWS133947 B
- JP H07318551 B
- JP 2007069193 A

**Non-patent literature cited in the description**

- **N. TANAKA ; H. KOBAYASHI ; K. NAKANISHI ; H. MINAKUCHI ; N. ISHIZUKA.** *Anal. Chem.,* 2001, vol. 73, 420-429
- **O. NUNEZ ; K. NAKANISHI ; N. TANAKA.** *J. Chromatogr. A,* 2008, vol. 1191, 231-252
- **R. E. PAPROSKI ; J. COOLEY ; C. A. LUCY.** *Analyst,* 2006, vol. 131, 422-428